# EUROPEAN PATENT APPLICATION

(11) **EP 4 094 732 A1**
(43) Date of publication of application: **30.11.2022**
(21) Application number: 22175860.0
(22) Date of filing: 27.05.2022
(51) Int. Cl.: A61F 5/01

(54) **HIP BRACE**

(30) Priority: 27.05.2021 IT 202100013847
(71) Applicant: Plus Biomedicals S.r.l., 25125 Brescia (IT)
(72) Inventor: MORA, Simone, 25125 Brescia (IT); VAVASSORI, Francesco, 25125 Brescia (IT)
(74) Representative: Chimini, Francesco

(57) **Abstract**

A hip brace (1; 100) according to the present invention is wearable by a patient around the waist for hip joint prevention and rehabilitation after hip surgery. The brace comprises an elastic body (10), suitable for being worn horizontally around the waist, and a support zone (30) positioned on the body (10), suitable for engaging the hip. The brace is characterized in that the support zone (30) comprises a vertical band (38) suitable for providing hip support, and at least one inclined band (36, 37) extending from said vertical band (38). The bands are made of elastomeric material to support the hip joint, thus reducing the risks of post-operative injuries.

## Description

The present invention relates to the field of braces, and in particular, a proprioceptive hip brace for the prevention and rehabilitation of a patient's hip joint after hip operation.

Hip replacement surgery has been a very successful practice for years: the significant advantages of this procedure include pain relief and a greater ability to perform routine activities. Unfortunately, hip replacement surgery has some potential complications that, although rare, can occur. Among these there is the dislocation of the hip prosthesis, an event occurring in 2-3% of cases. The dislocation of the hip prosthesis can have different causes and can be related to different aspects which can depend on the patient, the doctor, the type of prosthesis or the operation.

The highest number of cases of hip prosthesis dislocation has been found for operations with posterior access rather than with lateral or frontal access. In fact, 75% to 90% of cases of dislocation occur posteriorly and for this reason the approach with posterior access could, theoretically, contribute to instability.

In fact, with posterior access the gluteus muscle is damaged during the operation. In particular, the gluteus minimus shows damage which causes a decrease in the volume of the muscle itself. The main consequence of this muscle modification is the increase in the forces acting on the femur which has undergone the prosthesis operation. This phenomenon has been mainly found in the movement of getting up and sitting down from a chair due to the high involvement of the gluteus muscle group in such a type of movement.

There are several hip braces in the background art. The typical feature of the known braces is the presence of a metal alloy structure which wraps the pelvis and hip joint around. There are also fabric braces, provided with elastic bands, but still supported by metal slats placed sideways.

A well-known disadvantage of this solution is that the structures are bulky, so that in some cases they cannot be worn autonomously.

The problem to be solved is to provide a brace that eliminates the disadvantages of the braces of the prior art as reported above.

Such an object is achieved by a hip brace according to claim 1. The dependent claims describe preferred or advantageous embodiments of the invention.

The features and advantages of the wearable hip brace according to the invention will however become apparent from the following description of preferred embodiments thereof, given by way of non-limiting indication, with reference to the accompanying drawings, in which:
- figure 1 shows a rear view of the hip brace of the present invention, in accordance with an embodiment;
- figure 2 shows a side view of the hip brace in figure 1;
- figure 3 shows a plan view of the hip brace of the present invention, in a further embodiment;
- figure 4 shows a detail of the hip brace of the present invention, and in particular a support zone, in an alternative embodiment;
- figure 5 shows the positioning of the support zone, in an embodiment variant, with respect to the gluteus muscle, in a side view;
- figure 6 shows the positioning of the support zone in figure 5, in a rear view;

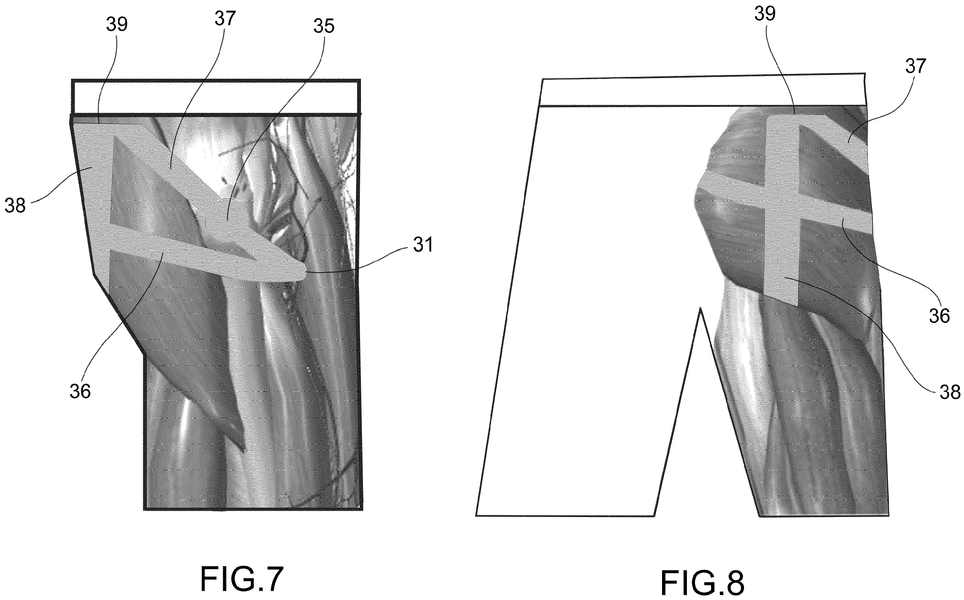

- figure 7 shows the positioning of the support zone, in a further embodiment variant, with respect to the gluteus muscle, in a side view;
- figure 8 shows the positioning of the support zone in figure 7, in a rear view;
- figures 9 and 9a show, in rear view and in front view, respectively, a hip brace according to a further embodiment; and
- figure 10 depicts a planar extension of the hip brace in figures 9 and 9a.

### DETAILED DESCRIPTION

With reference to the abovementioned figures, reference numeral 1 has been used to identify, as a whole, a proprioceptive hip brace for the prevention and rehabilitation of a patient's hip joint after hip surgery.

According to the invention, the hip brace 1 is wearable and comprises an elastic body 10 suitable for being worn horizontally around the patient's waist.

Preferably the body 10 is made of fabric, thus being advantageously light. Furthermore, due to reduced thickness, it is possible to wear it under the usual daily clothing. Finally, since it is made of stretch fabric, it is easily wearable autonomously by the user.

According to the invention, the hip brace 1 comprises a support zone 30 fixed to the body 10 and suitable for engaging the patient's hip once the brace 1 has been worn. Preferably, the support zone 30 is fixed to the body 10 so that it does not come into contact with the patient's skin once the brace 1 has been worn.

Preferably, the support zone 30 is a screen-printed silicone structure. As noted in the figures, the support zone 30 comprises a plurality of bands 36,37,38 suitable for providing hip support. Said bands 36,37,38 are made of elastomeric material, preferably of silicone.

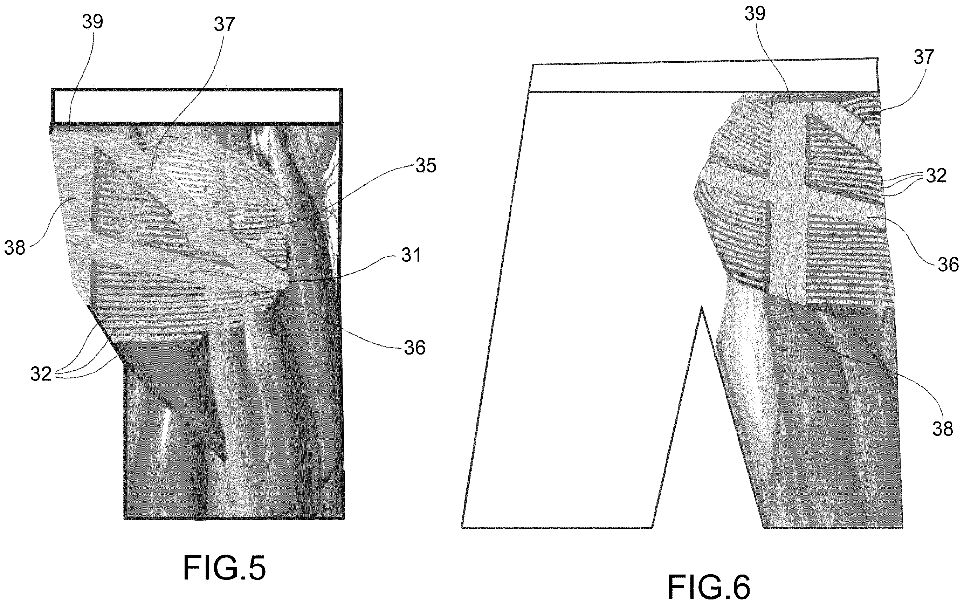

According to the invention, the support zone 30 comprises at least one vertical band 38 suitable for providing hip support. As it can be seen in figure 6, once the brace is worn, the vertical band 38 is arranged vertically centrally with respect to the gluteus muscle.

The vertical band 38 comprises an upper end 39 and a lower end 33.

Advantageously, the vertical band 38 performs a support function and a proprioceptive function for the patient's hip. In fact, such a vertical band 38, as a result of the progressive elongation, stiffens providing the patient with "feedback" on the effort of the movement in progress. This "feedback" disincentives the patient to move in an uncontrolled manner, which would cause damage to the operated hip, and prevents further ever closer movements to the critical area for the hip. In summary, the vertical band 38 limits the movements - Range Of Motion - which can be harmful to the patient and which lead to the dislocation of the prosthesis. This function of the brace is particularly useful in the post-operative rehabilitation phase.

The support zone 30 further comprises at least one inclined band 36, 37 extending from said vertical band 38.

Said at least one inclined band is a first inclined band 36 extending between said upper end 39 and said lower end 33 of the vertical band 38, in a first direction.

Preferably, the first direction is towards the patient's hip, so that, when the brace 1 is worn, the first inclined band 36 engages the gluteus maximus. Such a first inclined band 36 also provides a limitation of the directional movement for the hip when worn. In other words, the brace with the first inclined band 36 mimics the main directionality of the gluteus maximus muscle and contributes to the proprioceptive function.

In an embodiment, said first inclined band 36 also extends in a second direction opposite to the first direction. Such further extension of the first inclined band 36 allows further directional limitation of the hip.

Said at least one inclined band is a second inclined band 37 extending from said upper end 39 of the vertical band 38, in a first direction.

Preferably, the first direction is towards the patient's hip, so that, when the brace 1 is worn, the second inclined band 37 engages the gluteus minimus and contributes to the proprioceptive function of the hip. Such a second inclined band 37 also provides a limitation of directional movement for the hip when worn. In other words, the brace with the second inclined band 37 mimics the main directionality of the gluteus minimus muscle and contributes to the proprioceptive function of the hip.

In an embodiment, said first inclined band 36 and said second inclined band 37 intersect each other defining, together with the vertical band 38, a triangular support structure 30. Such a triangular shape allows greater support and allows mimicking the directionality of the muscle fibers of a healthy hip. Advantageously, this allows reducing the risks of post-operative injuries.

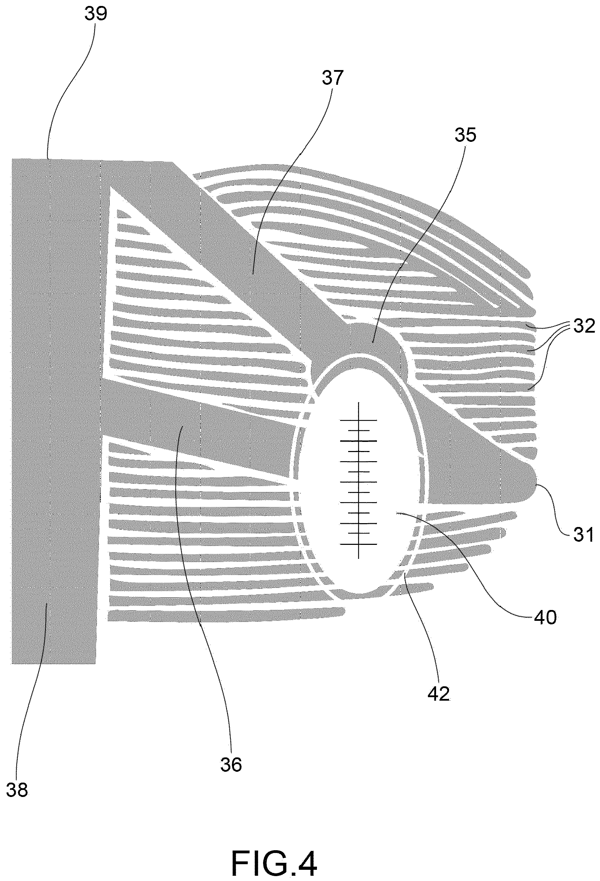

In an embodiment, the second inclined band 37 comprises an enlarged zone 35 arranged between an upper end 39 and a lower end 31 of the second inclined band 37. Preferably, said enlarged zone 35 is arranged close to the lower end 31 of the second inclined band 37. Figure 4 shows an example of position of the enlarged zone 35.

Preferably, the enlarged zone 35 is substantially circular. In further embodiments, said enlarged zone 35 is square, pentagonal, hexagonal, or any shape suitable for engaging the hip.

Advantageously, the enlarged zone 35, also made of silicone, has the dual function of supporting and identifying the anatomical landmark of the large trochanter to wear the brace correctly.

In an embodiment, shown for example in figure 4, the support zone 30 comprises support lines 32 arranged at least partially around said inclined bands 36, 37. The support lines 32 are also made of elastomeric material.

Such support lines 32 are smaller in size, i.e., thinner, than the bands 36,37,38 so that they do not restrict the movement of the patient and do not add simultaneously support to each movement.

Such support lines 32 mimic the directionality of the gluteus maximus and gluteus minimus and add to the proprioceptive function of the hip.

Preferably, the support lines 32 are substantially horizontal, at least below and above said first inclined band 36. As it can be seen in figure 4, the support lines are substantially horizontal also within the space defined by the triangular shape of the support structure 30.

Preferably, at least one part of the support lines 32 is substantially curved, at least in an upper end zone above the second inclined band 37.

In an embodiment, the support zone 30 comprises an insert 40 at least partially arranged on the inclined band 36,37.

The insert 40 is made of breathable stretch material. Advantageously, the breathable material with which the insert 40 is made of promotes breathability and therefore healing.

In an embodiment, the insert 40 has a silicone outer perimeter 42, which allows tensioning the skin and further promoting healing. Furthermore, such a solution prevents the formation of scars and maintains traction in the access route.

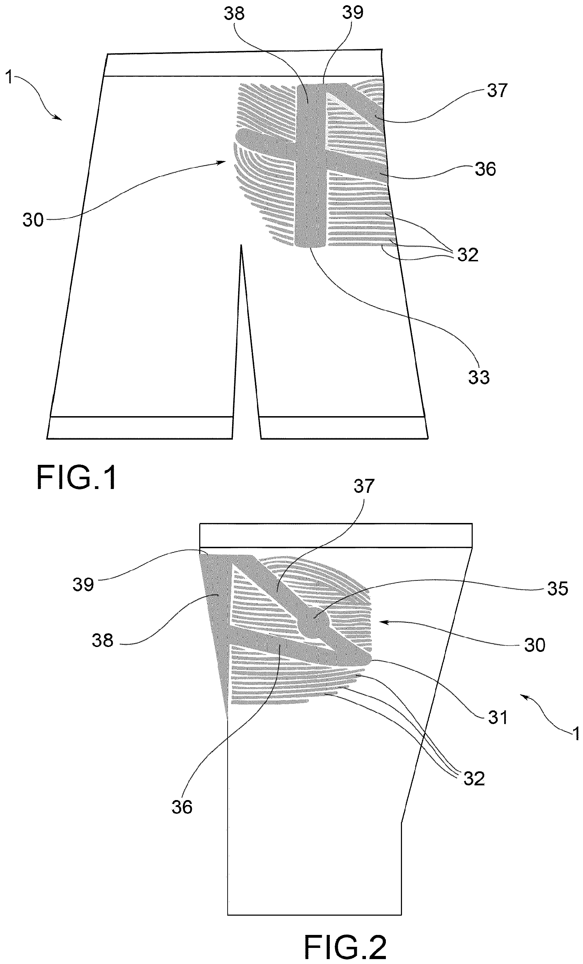

In an embodiment, said body 10 is integrated into shorts, as shown in figures 1 and 2.

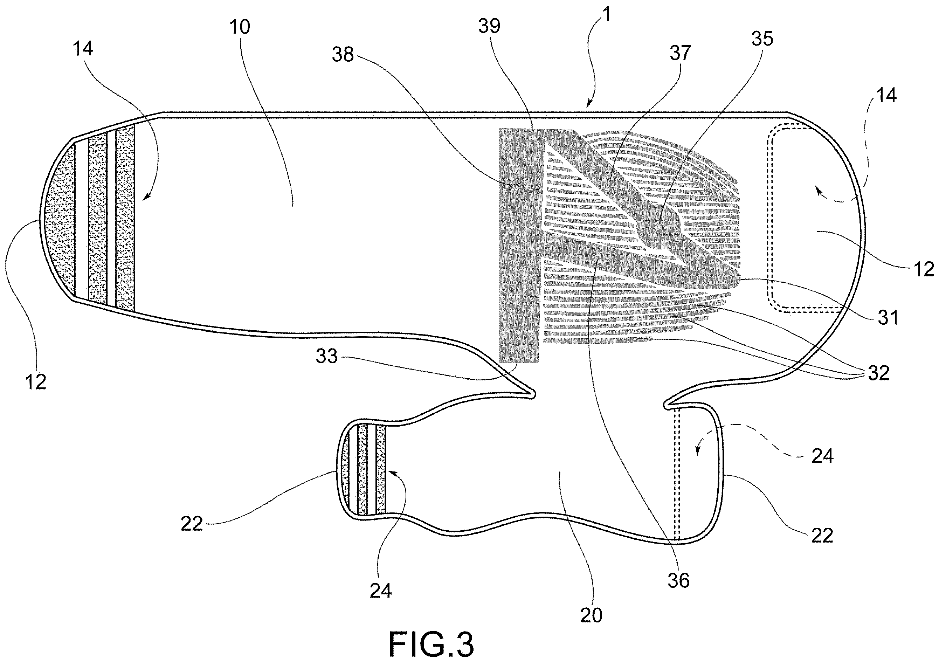

In an embodiment, said body 10 is an upper band, as shown in figure 3. In such an embodiment, the body 10 is provided with at least two opposite upper ends 12, each comprising upper attachment means 14 suitable for fixing the upper ends 12 to each other in a removable manner to allow the brace to be tied to the patient's waist.

In an embodiment, shown in figure 3, the brace 1 comprises a lower horizontal band 20 at least partially connected to the upper horizontal band 10. The lower horizontal band 20 is suitable for being tied horizontally to a patient's thigh. Such a lower band 20 comprises at least two opposite lower ends 22, each provided with lower attachment means 24 suitable for fixing the lower ends 22 to each other in a removable manner to allow the lower band 20 to be tied horizontally and removably to the patient's thigh.

In an embodiment, shown in figure 3, the upper horizontal band 20 and the lower horizontal band 24 are made in a single piece.

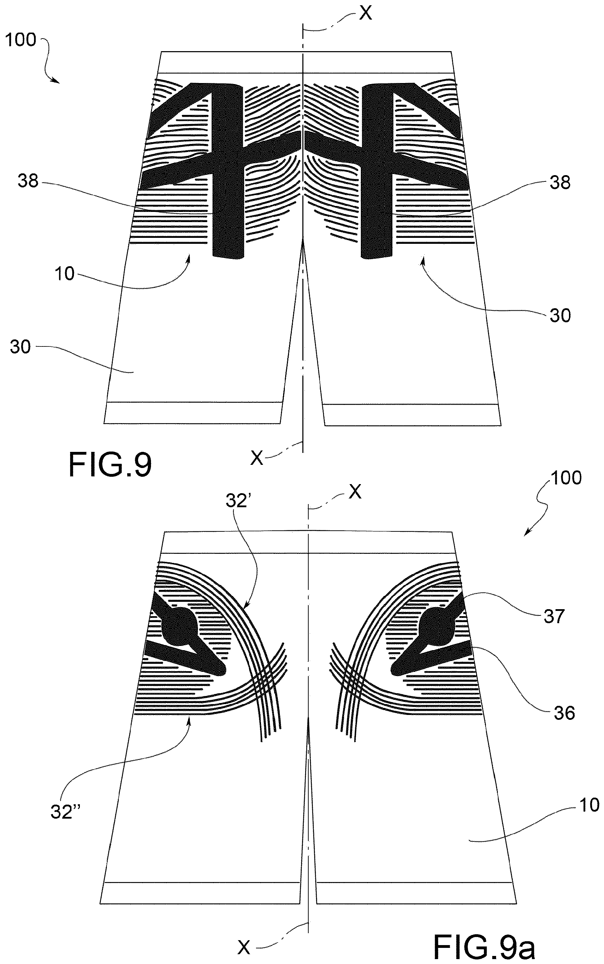

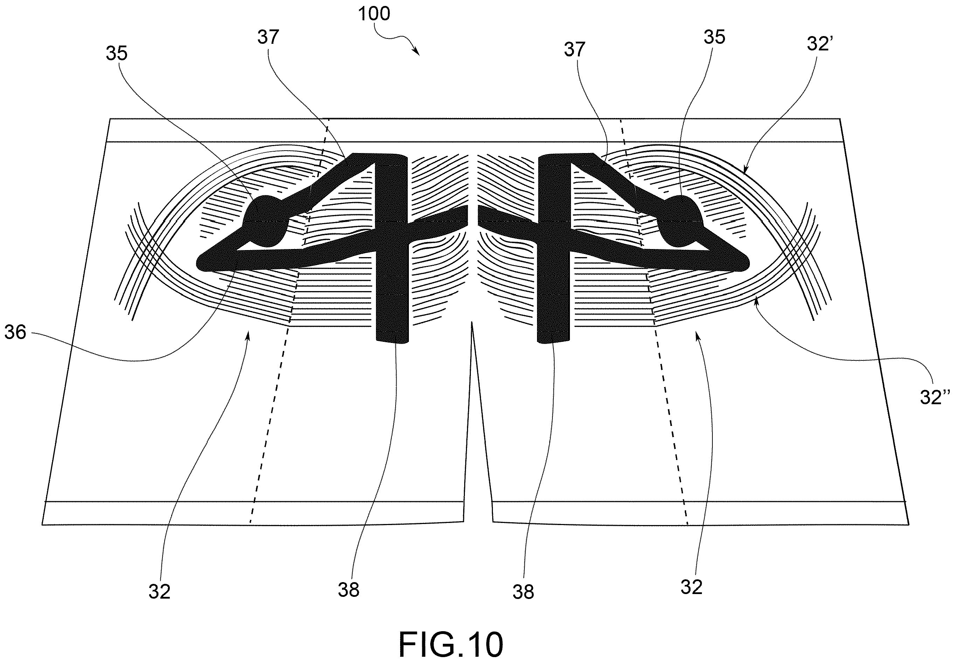

Figures 9, 9a and 10 show a hip brace 100 according to a further embodiment, for example made in the form of shorts. In this embodiment, the elements common to the embodiments described above are indicated with the same reference numerals.

With respect to the embodiments described above, the hip brace 100 is provided with two support zones 30, obtained specularly with respect to a vertical symmetry axis X of the elastic body 10.

Each support zone 30 can be made according to one, or a combination, of the above-described support zone 30 embodiments.

Therefore, the elastomeric support bands 36, 37, 38, 32 are bilateral, i.e., they are present, specularly, on both the side of the garment suitable for engaging the operated hip and the side of the garment suitable for engaging the healthy hip.

This bilateral configuration increases stability and makes the support more balanced. This bilaterality is based on the theory of mirror neurons applied to the contralateral operated limb - healthy limb pair.

In an embodiment (not necessarily related to the brace with two specular support zones 30), an upper portion 32' of the support lines 32 above the second inclined band 37, consisting of curved support lines, and a lower portion 32" of the support lines 32 below the first inclined band 36, consisting of by curved support lines, frontally elongate, mutually intersecting and forming a dovetail-shaped structure suitable for being placed at the femoral triangle zone.

This arrangement of the support lines 32', 32" is aimed at acting on major femoral blood vessels and promoting venous return.

Innovatively, a proprioceptive hip brace for the prevention and rehabilitation of the hip joint according to the present invention, is suitable for supporting a patient's hip joint after hip surgery, reducing the risks of post-operative injuries.

By virtue of the presence of silicone bands, the brace performs the support function of the gluteus muscle injured during the operation and helps in carrying out daily actions. The support provided by the brace can help the patient gain confidence in performing daily actions.

Advantageously, the brace reproduces the functions of the gluteus maximus muscle, performs a supportive function and helps the patient understand their own limits in movements.

Furthermore, by virtue of the presence of the silicone bands, the brace performs a support function of the gluteus muscle injured during the operation and helps to rebalance the forces acting on the hip joint mitigating the lameness, characteristic of patients who have undergone hip joint surgery.

Advantageously, moreover, the hip brace performs a constricting vessel function not only to promote blood circulation but also to perform a containment function.

Table T1 below shows the data of maximum absolute power released in the exercise of the squat jump by healthy subjects, without and with the hip brace according to the invention, detected by D-Wall instrumentation of the company TecnoBody^{®}.

**T1**

| | **p1** | **p2** | **p3** | **p4** | **p5** | **p6** | **p7** | **p8** | **p9** | **p10** |
|---|---|---|---|---|---|---|---|---|---|---|
| **Absolute power (W) without Brace** | 3338 | 2163 | 1618 | 2165 | 3867 | 3359 | 3863 | 4191 | 3811 | 2967 |
| **Absolute power (W) with Brace** | 3430 | 2153 | 1633 | 2235 | 4362 | 3125 | 4188 | 4594 | 3955 | 3313 |
| Difference % | 2.8 | -0.5 | 0.9 | 3.2 | 12.8 | -7.0 | 8.4 | 9.6 | 3.8 | 11.7 |

Eight out of ten subjects released more power with the hip brace on, with an average of +4.6%. Among these eight subjects the minimum variation was +1% and the maximum variation was +13%, while between the two subjects who released less power the decrease was -0.5% and -7%.

Table T2 below shows the stability index detected by a bipodal balance test performed on TechnoBody^{®} ProKin instrumentation; a low value indicates greater stability.

**T2**

| | **p1** | **p2** | **p3** | **p4** | **p5** | **p6** | **p7** | **p8** | **p9** | **p10** |
|---|---|---|---|---|---|---|---|---|---|---|
| **Stability index (°) without Brace** | 2.06 | 0.96 | 0.85 | 0.89 | 2.49 | 1.31 | 1.81 | 2.79 | 1.2 | 1.92 |
| **Stability index (°) with Brace** | 1.65 | 0.71 | 0.94 | 1.22 | 1.36 | 1.16 | 1.77 | 1.89 | 0.99 | 1.08 |
| Difference % | 19.9 | -26.0 | 10.6 | 37.1 | -45.4 | -11.5 | -2.2 | -32.3 | -17.5 | -43.8 |

In eight out of ten subjects (healthy, unoperated subjects) the stability index was lowered when the subject was wearing the brace. In the ten subjects, the average change in the stability index was -15%; the decrease in the index was from a minimum of -2% to a maximum decrease of -45%, while the increase in the stability index in the remaining two subjects was +10% and +37%.

In order to meet specific needs, those skilled in the art could make variations to the embodiments of the aforesaid hip brace or replace elements with functionally equivalent ones. Such variations also fall within the scope of protection as defined by the following claims. Furthermore, each variation described as belonging to a possible embodiment may be implemented regardless of the other variations described.

## Claims

1. A hip brace (1; 100) wearable by a patient around the waist for hip joint prevention and rehabilitation after hip surgery, comprising:
- an elastic body (10), suitable for being worn horizontally around the waist;
- a support zone (30) positioned on the body (10), suitable for engaging the hip;
wherein the support zone (30) comprises a vertical band (38) suitable for providing hip support, and at least one inclined band (36, 37) extending from said vertical band (38), and wherein said bands are made of elastomeric material.

2. A hip brace (1; 100) according to claim 1, wherein the vertical band (38) comprises an upper end (39) and a lower end (33), and wherein said at least one inclined band is a first inclined band (36) extending between said upper end (39) and said lower end (33) of the vertical band (38), in a first direction.

3. A hip brace (1; 100) according to claim 2, wherein said first inclined band (36) also extends in a second direction opposite to the first direction.

4. A hip brace (1; 100) according to any preceding claim, wherein said at least one inclined band is a second inclined band (37) extending from said upper end (39) of the vertical band (38).

5. A hip brace (1; 100) according to claim 4, wherein the second inclined band (37) comprises an enlarged zone (35) arranged between an upper end (39) and a lower end (31) of said second inclined band (37).

6. A hip brace (1; 100) according to claim 5, wherein said enlarged zone (35) is arranged close to said lower end (31) of said second inclined band (37).

7. A hip brace (1; 100) according to any one of claims 4 to 6, wherein said first inclined band (36) and said second inclined band (37) intersect each other thus forming, together with said vertical band (38), a triangular support zone (30).

8. A hip brace (1; 100) according to any one of the preceding claims, wherein said support zone (30) comprises support lines (32) arranged at least partially around said at least one inclined band (36,37), and wherein said support lines (32) are made of elastomeric material.

9. A hip brace (1; 100) according to the preceding claim, wherein the support lines (32) comprise an upper portion (32') obtained above the second inclined band (37) and consisting of curved support lines, and a lower portion (32") obtained below the first inclined band (36) and consisting of curved support lines, wherein said upper (32') and lower (32") portions elongate at the front, mutually intersecting and forming a dovetail-shaped structure suitable for being placed at the femoral triangle zone.

10. A hip brace (1; 100) according to any one of the preceding claims, wherein said support zone (30) comprises an insert (40) arranged at least partially on said at least one inclined band (36,37), and wherein said insert (40) is made of breathable elastic material.

11. A hip brace (1; 100) according to claim 10, wherein said insert (40) is provided with an outer perimeter (42) made of elastomeric material.

12. A hip brace (100) according to any one of the preceding claims, comprising two support zones (30) obtained specularly with respect to a vertical symmetry axis (X) of the elastic body (10) so as to be suitable for engaging the operated hip and the healthy hip, respectively.

13. A hip brace (1; 100) according to any preceding claim, wherein said elastic body (10) and said support zone (30) are integrated into a pair of shorts.

14. A hip brace (1) according to any one of claims 1-12, wherein said elastic body (10) is an upper strap which comprises at least two opposite upper ends (12), each comprising upper attachment means (14) suitable for fixing said upper ends (12) to each other in a removable manner to allow the upper strap to be tied to the patient's waist.

15. A hip brace (1) according to claim 14, wherein said elastic body (10) further comprises a lower strap (20) at least partially connected to the upper strap, comprising two opposite lower ends (22), each provided with lower attachment means (24) suitable for fixing said lower ends (22) to each other in a removable manner to allow the lower strap (20) to be tied to the patient's thigh.
